# EUROPEAN PATENT APPLICATION

(11) **EP 4 191 602 A1**
(43) Date of publication of application: **07.06.2023**
(21) Application number: 21858470.4
(22) Date of filing: 06.07.2021
(51) Int. Cl.: G16H 20/60, G16H 50/30, G16H 50/20, G16H 80/00, G06Q 50/10, G06Q 50/12

(54) **BIOMETRIC INFORMATION MANAGEMENT METHOD BASED ON LOCATION INFORMATION OF USER**

(30) Priority: 19.08.2020 KR 20200104192
(71) Applicant: i-Sens, Inc., Seoul 06646 (KR)
(72) Inventor: PARK, Jeong Je, Seoul 06646 (KR)
(74) Representative: BCKIP Part mbB
(86) International application number: PCT/KR2021/008590
(87) International publication number: WO 2022/039383

(57) **Abstract**

The present disclosure relates to a biometric information management method based on location information of a user, and more specifically, to a biometric information management method in which a restaurant where a user is located is determined on the basis of location information of the user, and calorie intake of the user is determined on the basis of the calories of each food menu provided at the restaurant, and thus the calorie intake of the user may be accurately determined, and changes in biometric information may be accurately determined on the basis of the calorie intake.

## Description

### TECHNICAL FIELD

The present disclosure relates to a method for managing biometric information based on location information of a user, and more specifically, a method for managing biometric information, the method which can accurately determine the calories or nutrients consumed by a user by determining a restaurant where a user is located based on location information of the user and determining intake calories or intake of the user nutrients based on calories and nutrients for each food menu sold by the restaurant and, based on this, can precisely determine changed biometric information.

### BACKGROUND

Diabetes is a major cause of death and a cause of disability worldwide, and therefore, many people have health problems due to diabetes. Specially, diabetes is a serious disease that causes heart and kidney disease, blindness, nerve damage and high blood pressure. According to a long-term clinical study, the incidence of complications can be significantly reduced by appropriately managing blood glucose levels. Therefore, it is important to continuously manage diabetes, an important factor is self-monitoring of blood glucose levels.

In response to this demand, a self-diagnosis biometer in which a user can check a blood glucose level of the user by himself or herself has been widely distributed and used. A conventional blood glucose meter measures the blood glucose level of the user by putting the user's blood on a sensor strip, which is a test strip. Accordingly, the sensor strip with the blood is inserted into the blood glucose meter, and the blood glucose level measured through the sensor strip is displayed on the blood glucose meter.

At this time, the blood glucose meter receives an electrical signal generated by an electrochemical reaction between the collected blood and the reactant in the sensor strip, and measures the blood glucose level. Such a blood-collect-type blood glucose meter (finger prick method) helps diabetic patients to manage blood glucose, but it is difficult to accurately identify the blood glucose levels which are being frequently changed because it shows only the result at the time of the measurement.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

To overcome such a drawback, continuous glucose monitoring systems (CGMSs) inserted into the human body to measure a blood glucose level every few minutes have been developed, and therefore easily perform the management of diabetics and responses to an emergency situation.

A continuous blood glucose measurement system includes a body attachable unit configured to be insertable into a human body to measure blood glucose by collecting a body fluid such as blood of a user, and a communication terminal for communicating with the body attachable unit and displaying a blood glucose level measured by the body attachable unit.

The body attachable unit includes a sensor that is partially inserted into the body and generates a biosignal representing the user's blood glucose value from the body fluid, and a transmitter that processes the biosignal received from the sensor, measures the blood glucose value of the user, and transmits blood glucose information including the measured blood glucose value to a communication terminal.

Blood glucose is greatly influenced by factors such as the type of food and the amount of food consumed by the user, the user can check the user's blood glucose in real time through a continuous blood glucose measurement system, and based on the value of the blood glucose changed according to the food eaten, the amount of meal may be adjusted or medications such as insulin may be administered.

The user needs to control the type of food or the amount of food consumed in order to manage the user's blood glucose level, and in order to efficiently and accurately manage the user's blood glucose level, the user must know the user's blood glucose change according to the amount of food consumed or the user's blood glucose change according to the type of food.

However, when the user dines at a restaurant, it is difficult to determine the exact calories of the food sold by the restaurant, and even if it is the same food menu, the same food menu has different calories depending on the restaurant, and therefore there is a problem in that it is difficult in accurate blood glucose control.

In addition, whenever the user dines at a restaurant, it is difficult for the user to identify and input the type of food and calories consumed by the user after each meal, and because of this, there is a problem in that it is difficult to determine the change in blood glucose of the user according to the meal at the restaurant.

### SUMMARY

### Technical Problem

The present disclosure is invented to solve problems in conventional blood glucose management methods, and the purpose of the present disclosure is for providing a method of, after checking a restaurant where a user is located based on location information of the user, managing biometric information changed according to food menu consumed by the user at the restaurant.

Another purpose of the present invention is for providing a method for accurately obtaining food information consumed at a restaurant where a user is located based on location information of the user and, based on this, managing biometric information.

Another purpose of the present invention is for providing a method for determining whether a user has finished dining at a restaurant based on a change in location information of the user, and managing biometric information of the user which is changed after the meal is finished.

Still another purpose to be achieved by the present disclosure is for providing a method for managing biometric information, the method in which whether a user currently exists in a restaurant based on location information of the user is determined, and if the user currently exists in the restaurant, whether the user has a meal is inquired so as to induce the user to input meal information without omission.

Still another object to be achieved by the present disclosure is for providing a method for managing biometric information, the method in which information on meal time for each user is preset, and whether the user currently exists in a restaurant only during the set meal time is inquired, thereby reducing the user's inconvenience caused by frequent inquiries.

### Solution to Problem

To accomplish the purpose of the present disclosure, a method for managing biometric information based on location information according to the present disclosure comprise: determining whether a user is currently located in a restaurant; when it is determined that the user is currently located in the restaurant, providing a menu list sold by the restaurant to the user and determining a type of food and calorie eaten by the user based on a food menu eaten by the user in the menu list; and providing the user with biometric information of the user which is changed according to the type of food and the calorie eaten by the user.

In an embodiment of the present disclosure, the determining of whether the user is currently located in the restaurant comprises determining whether the user is currently located in the restaurant based on restaurant information input through the user terminal.

In another embodiment of the present disclosure, the determining of whether the user is currently located in the restaurant comprises: determining location information of a position where the user is located; searching for restaurant information mapped to the location information, and providing the searched restaurant information to the user; and when a specific restaurant is selected among searched restaurants, determining that the user is currently located at the selected restaurant.

In still another embodiment of the present disclosure, the determining of whether the user is currently located in the restaurant comprises: determining location information of a position where the user is located; determining whether a restaurant mapped to the location information exists; when the restaurant mapped to the location information exists, outputting an inquiry message for inquiring whether the user is planning to dine to the user; when receiving a confirmation massage from the user in response to the inquiry message, searching for restaurant information mapped to the location information and providing the searched restaurant information to the user; and when a specific restaurant is selected among searched restaurants, determining that the user is currently located at the selected restaurant.

Preferably, a method for managing biometric information based on location information according to the present disclosure further comprises: determining whether a current time is within a time range set by the user, wherein when the current time is within the time range set by the user, the determining of whether the restaurant mapped to the location information exists is performed.

Preferably, according to the present disclosure, the determining of the type of food and the calorie eaten by the user comprises: searching for a menu list sold by a restaurant where the user is located; providing the searched menu list to the user, and determining a food menu selected by the user in the menu list as a food menu eaten by the user; and determining the type of food and the calorie eaten by the user by searching a food type identifier and calorie information related to the food menu eaten by the user.

In a method for managing biometric information based on location information according to the present disclosure, the food type identifier and the calorie information related to the food menu eaten by the user are provided from a restaurant providing the food menu and are stored in a database.

Preferably, a method for managing biometric information based on location information according to the present disclosure further comprises: determining whether current location information of the user is changed to be different from location information of the restaurant based on location information of the user; and when the current location information of the user is changed, determining that the user finishes dining, wherein biometric information of the user which is changed according to the type of food and the calorie eaten by the user at a time of finishing dining is determined.

Preferably, a method for managing biometric information based on location information according to the present disclosure further comprises: receiving a food menu identifier for which the user wants to search; searching for a restaurant selling food corresponding to the food menu identifier among restaurants close to a current location of the user; and providing information on the searched restaurant to the user.

Here, a method for managing biometric information based on location information according to the present disclosure further comprises: receiving a target calorie range together with the food menu identifier for which the user wants to search, searching for one or more restaurants located close to the current location of the user among restaurants which sell food satisfying the target calorie range and corresponding to the food menu identifier, and providing the searched one or more restaurants to the user.

### Advantageous Effects of Invention

A method for managing biometric information according to the present disclosure has various effects as follows.

First, a method for managing biometric information according to the present disclosure can accurately determine calories consumed by a user, and, based on this, precisely determine the changing biometric information by determining a restaurant where a user is located based on location information of the user, and determining calorie intake of the user based on the calorie of each food menu provided by the restaurant.

Second, a method for managing biometric information according to the present disclosure can manage biometric information of a user which changes after the meal is finished by determining whether the user has finished dining at a restaurant based on a change in location information of the user.

Third, a method for managing biometric information according to the present disclosure can induce a user to input meal information of the user without omission by determining whether the user currently exists in a restaurant based on location information of the user, and, if the user currently exists in the restaurant, inquiring whether the user has a meal.

Fourth, a method for managing biometric information according to the present disclosure can reduce the user's inconvenience caused by frequent inquiries by presetting information on meal time for each user, and inquiring whether the user currently exists in a restaurant only during the set meal time.

### BRIEF DESCRIPTION OF THE DRAWINGS

FIG. 1 is a diagram for explaining a system for managing biometric information according to an embodiment of the present disclosure.
FIG. 2 is a functional block diagram for explaining an apparatus for managing blood glucose information according to an embodiment of the present disclosure.
FIG. 3 is a functional block diagram for explaining an example of a food intake determination unit according to an embodiment of the present disclosure.
FIG. 4 is a functional block diagram for explaining an example of a meal determination unit according to an embodiment of the present disclosure.
FIG. 5 is a functional block diagram for explaining another example of a meal determination unit according to an embodiment of the present disclosure.
FIG. 6 is a flowchart for illustrating a method of managing blood glucose information according to an embodiment of the present disclosure.
FIG. 7 is a flowchart for illustrating an example of a method of determining whether a user is currently located at a restaurant in an embodiment of the present disclosure.
FIG. 8 is a flowchart for illustrating another example of determining whether a user is currently located at a restaurant in an embodiment of the present disclosure.
FIG. 9 is a flowchart for illustrating an example of providing meal information to a user according to an embodiment of the present disclosure.
FIG. 10 illustrates an example of an interface screen provided to a user terminal.
FIG. 11 illustrates an example of an interface screen for inquiring whether a user is planning to dine.
FIG. 12 illustrates an example of an interface screen for displaying a meal time range set by a user.
FIG. 13 illustrates an example of a blood glucose change report provided to a user.
FIG. 14 is a flowchart for illustrating a method of recommending a restaurant or food to a user.

### DETAILED DESCRIPTION OF EMBODIMENTS

The technical terms used in the present disclosure are only for the purpose of describing exemplary embodiments, and they are not intended to limit the present invention. Also, unless otherwise defined, all technical terms used herein should be construed as having the same meaning as commonly understood by those skilled in the art, and should not be interpreted as being excessively inclusive or excessively restrictive. In addition, when a technical term used herein is an erroneous technical term that does not accurately represent the idea of the present invention, it should be understood as replacing the term by a technical term which can be properly understood by those skilled in the art.

Further, singular expressions used in the present specification include plural expressions unless they have definitely opposite meanings. In the present application, it shall not be construed that terms, such as "including" or "comprising", various constituent elements or steps described in the specification need to be all essentially included, and it shall be construed that some constituent elements or steps among the various constituent elements or steps may be omitted, or additional constituent elements or steps may be further included.

Also, it should be noted that the accompanying drawings are merely illustrated to easily explain the spirit of the invention, and therefore, they should not be construed to limit the spirit of the invention by the accompanying drawings.

Hereinafter, a method for managing biometric information according to the present disclosure will be described in more detail with reference to the accompanying drawings.

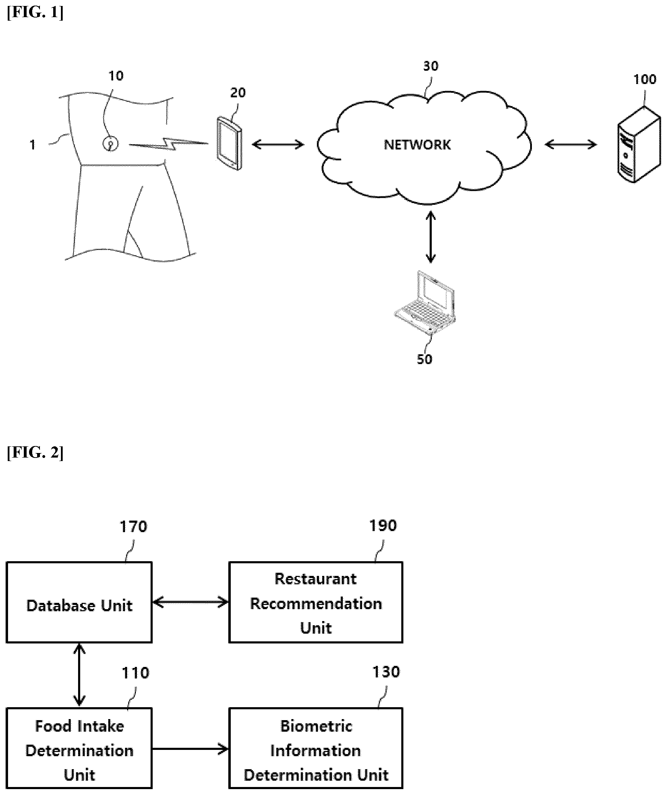

FIG. 1 is a diagram for explaining a system for managing biometric information according to an embodiment of the present disclosure.

Referring to FIG. 1, a user terminal (20), a restaurant terminal (50), and a management server (100) are connected to a network (30). Here, various types of wired or wireless networks capable of transmitting and receiving data between the user terminal (20), the restaurant terminal, and the management server (100) may be used to implement the network (30).

Meanwhile, a body attachable unit (10) is attached to a part of the user's body to continuously measure the user's biometric information, and transmits the measured biometric information to the user terminal (20) through a wired or wireless type communication.

The body attachable unit (10) may measure various biometric information of the user, but an example of the biometric information will be described below as blood glucose information.

The user terminal (20) includes a location information acquiring unit such as a GPS module and the like capable of acquiring location information of the user, and transmits information of current location of the user or blood glucose information of the user to the management server (100) through the network (30).

Meanwhile, the restaurant terminal (50) is a terminal used by an operator or manager who operates a restaurant, and the restaurant terminal (50) transmits, to the management server (100), restaurant information such as location information, a trade name, and a manager of the restaurant, food menus sold by the restaurant, and food menu information such as calories and nutrients of each food menu. Preferably, food menus have preset identifiers according to food types, and the restaurant terminal (50) may transmit information on food menus sold by the restaurant as food type identifiers.

The management server (100) classifies the restaurant information and the information on food menus sold by the restaurant and calories or nutrients for each food menu, received from the restaurant terminal (50), by restaurant, and registers and stores them in a database unit, and when receiving the location information from the user terminal (20), the management server (100) may determine which restaurant the user has currently visited based on the location information, provide the user terminal (20) with a menu list sold by the visited restaurant and information on calories and nutrients for each food menu, and provide the user terminal (20) with blood glucose information that is changed according to the food which the user ingests.

Depending on the field to which the present disclosure is applied, the management server (100) determines which restaurant the user is currently located based on the location information of the user and provides the user terminal (20) with only the menu list provided by the restaurant and information on calories and nutrients for each food menu, and the user terminal (20) may determine blood glucose information that is changed according to the food consumed by the user based on the food menu consumed by the user himself or herself and blood glucose information.

Even if the same food menu is provided by a plurality of restaurants, the calories or nutrients of the same food menu may be different for each restaurant, or the actual change in the blood glucose level of the user when the user eats that food menu may be different, and the user can easily acquire and input information on food menus and calories or nutrients consumed at the restaurant which the user visits, and can easily determine blood glucose information that is changed depending on the food taken at the restaurant where the user visits.

FIG. 2 is a functional block diagram for explaining an apparatus for managing blood glucose information according to an embodiment of the present disclosure.

Referring to FIG. 2 in more detail, a food intake determination unit (110) determines whether the user is currently located at a restaurant based on the location information of the user, and generates meal information based on the food menu and calorie information which the user currently consumes at the restaurant. Here, the calorie information includes calories and nutrients of a food menu.

A biometric information determination unit (130) determines biometric information, such as blood glucose information, which is changed according to the food consumed by the user, based on the meal information. The biometric information determination unit (130) determines the blood glucose information, which is changed after meal time, based on the food information (such as the type of food consumed by the user, the amount of meal, calorie information, meal start time and meal end time, etc.) and the blood glucose information continuously measured in real time by the body attachable unit. Accordingly, the biometric information determination unit (130) generates a blood glucose change report including blood glucose information, which is changed after a meal, based on the meal information and the blood glucose information, and provides the generated report to the user.

Preferably, an apparatus for managing blood glucose information according to an embodiment of the present disclosure further includes a database unit (170) configured to store restaurant information and food information, and the food intake determination unit (110) can determine whether the user is currently located at a restaurant based on the restaurant information registered and stored in the database unit (170), or can determine the type of food and calorie information consumed by the user based on food menu information registered and stored in the database unit (170).

More preferably, an apparatus for managing blood glucose information according to an embodiment of the present disclosure further includes a restaurant recommendation unit (190), and the restaurant recommendation unit (190) recommends to the user one or more restaurants located adjacent to the user based on the location information of the user or searches for one or more restaurants selling a food menu, which is within a target calorie range of the user, among restaurants selling food menus that the user wants to eat and provides the search result to the user.

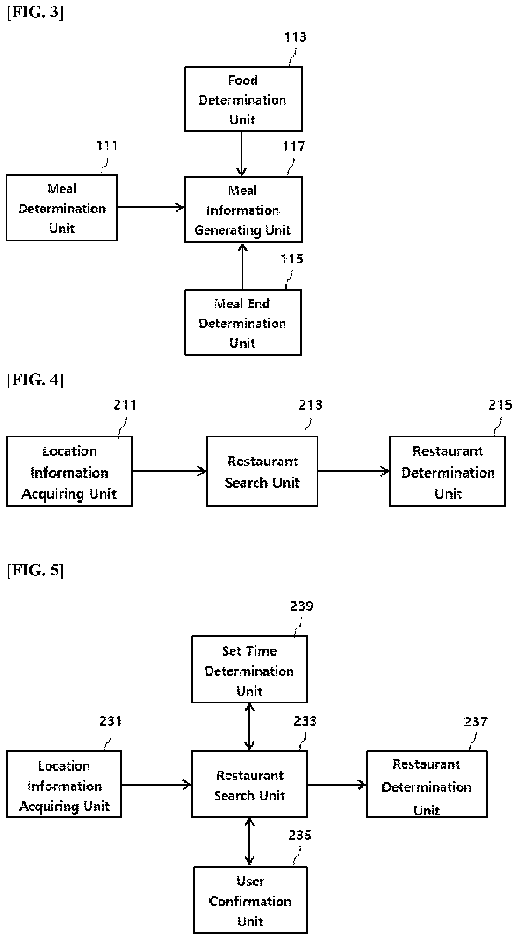

FIG. 3 is a functional block diagram for explaining an example of a food intake determination unit according to an embodiment of the present disclosure.

Referring to FIG. 3 in more detail, a meal determination unit (111) determines whether the user is currently located in a restaurant. The meal determination unit (111) may determine whether the user is currently located at the restaurant based on the restaurant name directly input by the user or determine whether the user is currently located at the restaurant based on the location information acquired through the user terminal.

A food determination unit (113) searches and retrieves a food menu list provided by a restaurant visited by the user from the database unit and provides it to the user, and determines a food menu consumed by the user and calorie information of the food menu based on the food menu selected by the user from the provided food menu list.

A meal end determination unit (115) determines whether the user has finished eating at the restaurant which the user visited, and for example, the meal end determination unit (115) determines that the meal has ended based on a meal end command directly input by the user or determines that the meal has ended when the user leaves the corresponding restaurant based on the location information of the user.

A meal information generating unit (117) is configured to, when it is determined that the user's meal has ended, generate meal information such as the name of the restaurant visited by the user, the food menu consumed at the visited restaurant, calorie information, total meal time and so on. Preferably, the meal information generating unit (117) may determine that the user has started eating at the time when it is determined that the user is currently located in the restaurant.

FIG. 4 is a functional block diagram for explaining an example of a meal determination unit according to an embodiment of the present disclosure.

Referring to FIG. 4 as a reference, a location information acquiring unit (211) acquires location information of the user, and a restaurant search unit (213) searches for a restaurant registered with location information of the restaurant matching the location information of the user in the database unit, and provides the searched restaurant information to the user. Here, the restaurant information provided to the user may be a restaurant name.

A restaurant determination unit (215) is configured to, when a specific restaurant is selected from the restaurant information provided to the user, determine that the user is currently located at the selected restaurant.

FIG. 5 is a functional block diagram for explaining another example of a meal determination unit according to an embodiment of the present disclosure.

Referring to FIG. 5 in more detail, a location information acquiring unit (231) acquires location information of the user, and a restaurant search unit (233) searches for a restaurant registered with location information of the restaurant matching the acquired location information of the user in the database unit.

A user confirmation unit (235) is configured to, if a restaurant registered with the location information of the restaurant that matches the location information of the user exists, generate an inquiry message for inquiring whether the user is currently planning to dine, and transmit the generated inquiry message to the user, and if receiving a confirmation message from the user in response to the inquiry message, determine that the user is currently planning to dine.

The restaurant search unit (233) is configured to, when it is determined that the user has visited a restaurant with a current meal plan, search for a restaurant registered with the same location as the location information of the user and provide the searched restaurant information to the user.

A restaurant determination unit (237) is configured to, when a specific restaurant is selected from the restaurant information provided to the user, determine that the user is currently located at the selected restaurant.

Preferably, a set time determination unit (239) is configured to determine whether the current time is within a meal time range set by the user, and a restaurant search unit (233) is configured to, when the current time is within the meal time range set by the user, determine whether there is a restaurant registered with location information of the restaurant matching the location of the user, or the user confirmation unit (235) is configured to, when the current time is within the meal time range set by the user and at the same time the restaurant registered with the location information of the restaurant matching the location information of the user is searched in the database, transmit an inquiry message for inquiring whether the user is currently planning to dine to the user.

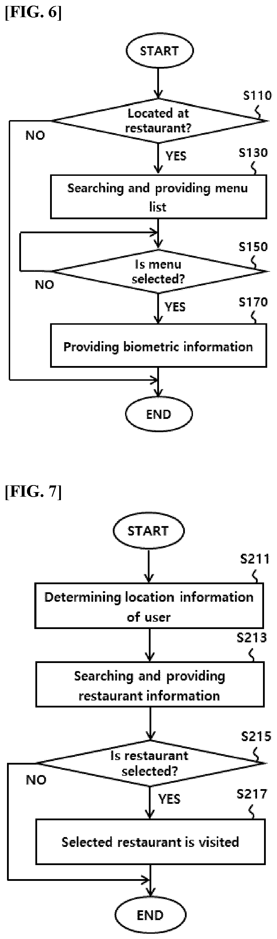

FIG. 6 is a flowchart for illustrating a method of managing blood glucose information according to an embodiment of the present disclosure.

Referring to FIG. 6 in more detail, whether the user is currently located in a restaurant is determined (5110).

If it is determined that the user is currently located at the restaurant, a list of food menus sold by the restaurant where the user is located and information on calories for each food menu are searched and retrieved from the database unit, and information on the list of the searched food menus and the searched calories for each food menu is provided to the user (S130).

When the user receives a user command for selecting a food menu to be consumed from the food menu list (S 150), blood glucose information of the user which is changed after ingesting the selected food menu is determined, and a blood glucose change report including the blood glucose information and food information is generated and provided to the user (S170).

FIG. 7 is a flowchart for illustrating an example of a method of determining whether a user is currently located at a restaurant in an embodiment of the present disclosure.

Referring to FIG. 7 in more detail, the location information of the user is acquired to determine the current location of the user (S211).

Restaurant information registered as location information matching the current location information of the user is searched in the database unit and the searched restaurant information is provided to the user (S213). The restaurant location information and trade name are registered and stored in the database unit, and a restaurant registered with location information matching the current location information of the user is searched.

When a specific restaurant is selected from the restaurant information provided to the user (S215), it may be determined that the user visits the selected restaurant and dines at the selected restaurant (S217).

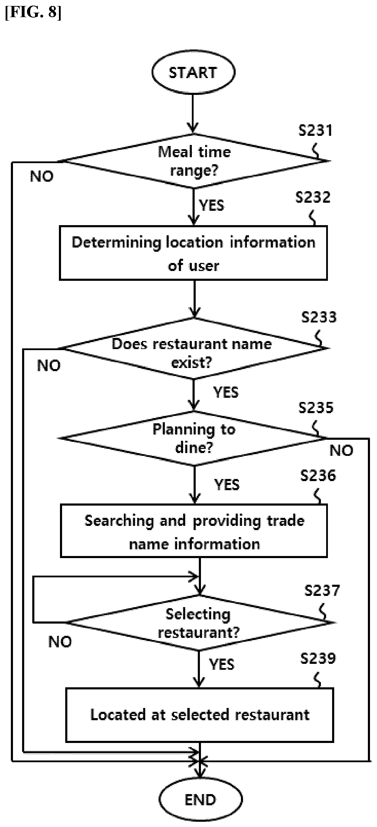

FIG. 8 is a flowchart for illustrating another example of determining whether a user is currently located at a restaurant in an embodiment of the present disclosure.

Referring to FIG. 8 in more detail, whether the current time is within a meal time range set by the user is determined (S231).

If the current time is within the meal time range set by the user, the location information of the user is acquired to determine the current location of the user (S232).

Whether a restaurant registered with location information matching the location information of the exists in the database unit is searched based on the location information of the user (S233).

If the current time is within the meal time range set by the user and at the same time a restaurant registered with location information matching the location information of the user exists in the database, whether the user is currently planning to dine is checked (S235). Preferably, an inquiry message for inquiring whether the user is currently planning to dine is generated and provided to the user, and when a confirmation message is received in response to the inquiry message from the user, it may be determined that the user is planning to dine.

When it is determined that the user is planning to dine, restaurant information registered with location information matching the current location information of the user is searched in the database unit, and the searched restaurant information is provided to the user (S236).

When a specific restaurant is selected from the restaurant information provided to the user (S237), it may be determined that the user visits the selected restaurant and dines at the selected restaurant (S239).

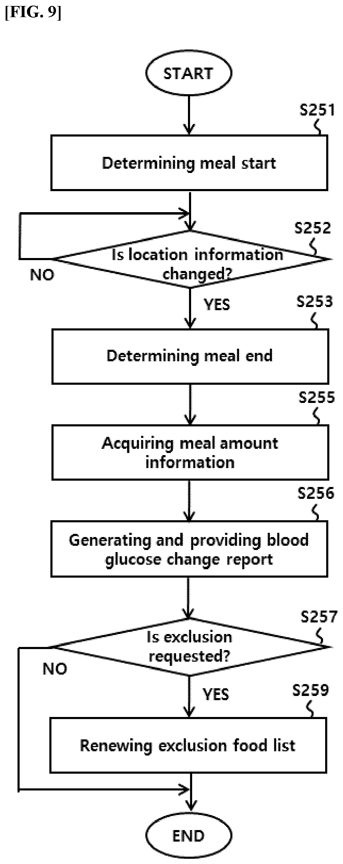

FIG. 9 is a flowchart for illustrating an example of providing meal information to a user according to an embodiment of the present disclosure.

Referring to FIG. 9 in more detail, a time when the user visits a restaurant and starts dining is determined (S251). Preferably, a meal start time of the user may be a time when it is determined that the user is located in the restaurant.

Based on the location information of the user, whether the location information of the user is changed is determined (S252), and when the location information of the user is changed from the restaurant to another location, it is determined that the user has finished dining (S253).

When the user has finished dining, a meal amount inquiry message for inquiring about the meal amount of the food menu selected by the user is generated and provided to the user, and the meal amount information of the food menu selected by the user is acquired based on a meal amount confirmation message received from the user in response to the meal amount inquiry message (S255).

A blood glucose change report is generated from a restaurant visited by the user, a food menu consumed at the restaurant, meal start time and meal end time, total dining time, calorie intake calculated based on meal amount information, and blood glucose information of the user changed due to the meal and is provided to the user (S256).

When a user command for requesting registration of the corresponding restaurant or food menu selected from the corresponding restaurant as an exclusion list is received from the user based on the blood glucose change report provided to the user (S257), the corresponding restaurant or the food menu selected at the corresponding restaurant is registered and renewed in the exclusion list of the user (S259).

If a restaurant or food menu is registered on the exclusion list, when the user later visits the restaurant on the exclusion list, an alarm for informing the user that the visited restaurant is a restaurant registered on the exclusion list may be provided, or an alarm for notifying that the food menu selected at the visited restaurant is registered on the exclusion list may be provided.

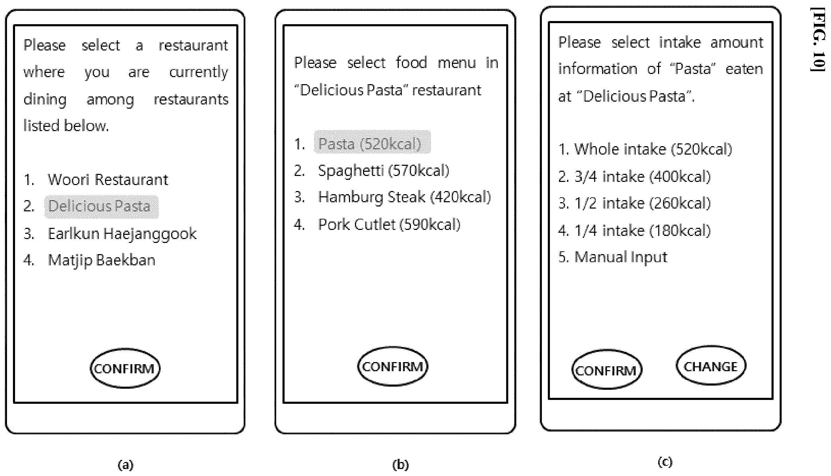

FIG. 10 illustrates an example of an interface screen provided to a user terminal.

As shown in FIG. 10 (a), based on the location information of the user, restaurant information registered with location information matching the location information of the user is searched and the searched restaurant information is provided to the user. When a specific restaurant is selected among the restaurants provided to the user, it is determined that the user has visited the selected restaurant.

As shown in FIG. 10(b), when a restaurant selected by the user is visited, a list of food menus sold by the selected restaurant and information on calories for each food menu are provided to the user terminal. When a specific food menu is selected from the food menu list provided to the user, it is determined that the user ate the selected food menu at the restaurant.

As shown in FIG. 10(c), when it is determined that the user has finished dining, the user terminal is provided with an interface screen for obtaining meal amount information of the food menu selected at the restaurant visited by the user with the user terminal. The user may select the meal amount of the selected food menu from among the meal amounts illustrated on the interface screen, or the user may directly input meal amount information.

Preferably, when the meal menu eaten by the user is changed in the middle or there is an added meal menu other than the meal menu selected by the user, the user can select a change icon to input the changed meal menu or add the added meal menu.

When the changed meal menu is input or a meal menu is to be added, a list of food menus sold by the visited restaurant and information on calories for each food menu may be provided to the user again.

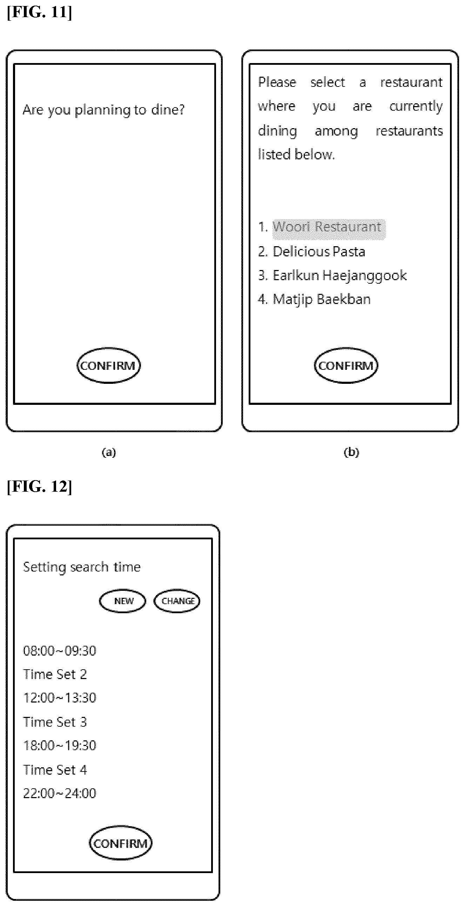

FIG. 11 illustrates an example of an interface screen for inquiring whether a user is planning to dine.

As shown in FIG. 11 (a), when a restaurant exists in a place where the user is located within a meal time range set by the user based on the current time and the location information of the user, an interface screen for inquiring whether the user is planning to dine is provided to the user terminal.

As shown in FIG. 11(b), when the user is planning to have a meal, information on restaurants registered with locations matching the location information of the user is provided to the user terminal.

FIG. 12 illustrates an example of an interface screen for displaying a meal time range set by a user.

As shown in FIG. 12, the user can set meal times divided into breakfast (time setting 1), lunch (time setting 2), dinner (time setting 3), and late-night snack (time setting 4).

Based on the user's eating habits, the user can freely register or change the meal times for each type of meal.

The meal time range is set in this way, and whether the user is planning to eat is asked to the user only when the current time is within the meal time range and at the same time a restaurant exists at the current location information of the user, thereby avoiding frequent meal schedule inquiries to the user and preventing omission of a meal information input.

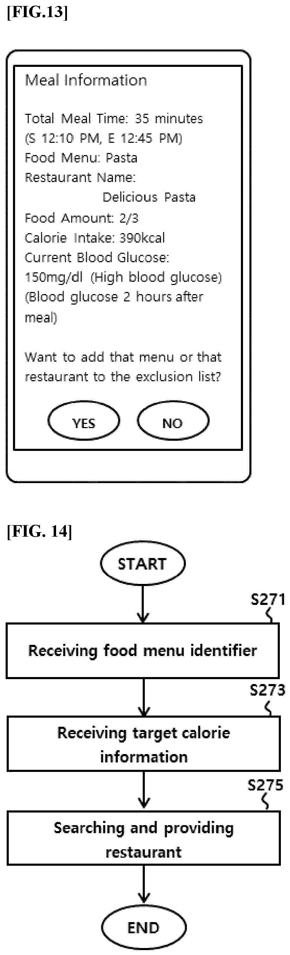

FIG. 13 illustrates an example of a blood glucose change report provided to a user.

As shown in FIG. 13, total meal time (meal start time and meal end time), meal menus eaten by the user, a visited restaurant name, a user's meal amount, calorie intake, and post-meal blood glucose information are provided.

Based on current blood glucose information after a certain amount of time has elapsed after the end of the meal, the user can confirm that the user's blood glucose level is high when eating the meal menu selected at the corresponding restaurant, and can add the selected restaurant or the meal menu consumed at the selected restaurant to the exclusion list.

Preferably, when the user's blood glucose is abnormal (e.g., high blood glucose) in the blood glucose change report, the biometric information determination unit (130) searches the database unit (170) for a history of eating the same food menu at the same restaurant and searches for a meal history in which a user's blood glucose changed after the meal is normal among the meal histories. The biometric information determination unit (130) may provide advice on meal information based on the meal history which is normal, when eating a food menu selected at a corresponding restaurant.

That is, the biometric information determination unit (130) compares total meal time, meal amount, and calorie intake of the same food menu at the same restaurant based on the previous meal history, which was normal, with total meal time, meal amount, and calorie intake of the same food menu at the current same restaurant, and can provide advice such as "eat slowly" or "reduce the amount of meal by half' based on the comparison result.

FIG. 14 is a flowchart for illustrating a method of recommending a restaurant or food to a user.

As shown in FIG. 14, a food menu identifier desired by the user is received from the user (S251). In addition, a target calorie range is received from the user together with the food menu identifier (S273).

One or more recommended restaurants located close to the user's current location are searched among restaurants that satisfy the target calorie range and sell food corresponding to the food menu identifier and are provided to the user (S275).

Preferably, when information on the recommended restaurants is provided to the user, it is possible to search for recommended restaurants by excluding restaurants or food menus registered in the exclusion list.

Meanwhile, the exemplary embodiments of the present disclosure described above can be implemented through programs executable at computers, and can be operated in a general-purpose digital computer executing the programs using computer readable medium.

The above-referenced computer readable medium comprises storage medium such as magnetic storage media (e.g., ROM, floppy disks, hard disks, etc.), optical recording media (e.g., CD-ROMs, DVDs, etc.), and carrier waves (e.g., transmission through the Internet).

The present disclosure has been described with reference to embodiments shown in the drawings, but this is only exemplary, and it will be understood by those skilled in the art that various modifications and other equivalent embodiments are possible therefrom. Accordingly, the technical protection scope of the present disclosure should be determined by the technical spirit of the appended claims.

## Claims

1. A method for managing biometric information based on location information, the method comprising:
determining whether a user is currently located in a restaurant;
when it is determined that the user is currently located in the restaurant, providing a menu list sold by the restaurant to the user and determining a type of food and calorie eaten by the user based on a food menu eaten by the user in the menu list; and
providing the user with biometric information of the user which is changed according to the type of food and the calorie eaten by the user.

2. The method for managing the biometric information based on the location information according to claim 1, wherein the determining of whether the user is currently located in the restaurant comprises determining whether the user is currently located in the restaurant based on restaurant information input through the user terminal.

3. The method for managing the biometric information based on the location information according to claim 1, wherein the determining of whether the user is currently located in the restaurant comprises:
determining location information of a position where the user is located;
searching for restaurant information mapped to the location information, and providing the searched restaurant information to the user; and
when a specific restaurant is selected among searched restaurants, determining that the user is currently located at the selected restaurant.

4. The method for managing the biometric information based on the location information according to claim 1, wherein the determining of whether the user is currently located in the restaurant comprises:
determining location information of a position where the user is located;
determining whether a restaurant mapped to the location information exists;
when the restaurant mapped to the location information exists, outputting an inquiry message for inquiring whether the user is planning to dine to the user;
when receiving a confirmation massage from the user in response to the inquiry message, searching for restaurant information mapped to the location information and providing the searched restaurant information to the user; and
when a specific restaurant is selected among searched restaurants, determining that the user is currently located at the selected restaurant.

5. The method for managing the biometric information based on the location information according to claim 4, further comprising
determining whether a current time is within a time range set by the user,
wherein when the current time is within the time range set by the user, the determining of whether the restaurant mapped to the location information exists is performed.

6. The method for managing the biometric information based on the location information according to any one of claims 2 to 4, wherein the determining of the type of food and the calorie eaten by the user comprises:
searching for a menu list sold by a restaurant where the user is located;
providing the searched menu list to the user, and determining a food menu selected by the user in the menu list as a food menu eaten by the user; and
determining the type of food and the calorie eaten by the user by searching a food type identifier and calorie information related to the food menu eaten by the user.

7. The method for managing the biometric information based on the location information according to claim 6, wherein the food type identifier and the calorie information related to the food menu eaten by the user are provided from a restaurant providing the food menu and are stored in a database.

8. The method for managing the biometric information based on the location information according to any one of claims 2 to 4, further comprising:
determining whether current location information of the user is changed to be different from location information of the restaurant based on location information of the user; and
when the current location information of the user is changed, determining that the user finishes dining,
wherein biometric information of the user which is changed according to the type of food and the calorie eaten by the user at a time of finishing dining is determined.

9. The method for managing the biometric information based on the location information according to any one of claims 2 to 4, further comprising:
receiving a food menu identifier for which the user wants to search;
searching for a restaurant selling food corresponding to the food menu identifier among restaurants close to a current location of the user; and
providing information on the searched restaurant to the user.

10. The method for managing the biometric information based on the location information according to claim 9, further comprising
receiving a target calorie range together with the food menu identifier for which the user wants to search,
searching for one or more restaurants located close to the current location of the user among restaurants which sell food satisfying the target calorie range and corresponding to the food menu identifier, and
providing the searched one or more restaurants to the user.
